# EUROPEAN PATENT SPECIFICATION

(11) **EP 1 891 901 B1**
(45) Date of publication and mention of the grant of the patent: **23.07.2014**
(21) Application number: 06757146.3
(22) Date of filing: 07.06.2006
(51) Int. Cl.: A61B 10/00, A61B 10/04

(54) **TISSUE TRAPPING APPARATUS, TREATMENT INSTRUMENT FOR ENDOSCOPE, AND ENDOSCOPE**
GEWEBEKLEMMVORRICHTUNG, BEHANDLUNGSINSTRUMENT FÜR ENDOSKOPE UND ENDOSKOP
APPAREIL DE CAPTURE DE TISSU, INSTRUMENT DE TRAITEMENT POUR ENDOSCOPE ET ENDOSCOPE

(30) Priority: 16.06.2005 JP 2005176499
(43) Date of publication of application: 27.02.2008
(73) Proprietor: OLYMPUS MEDICAL SYSTEMS CORP., Tokyo 151-0072 (JP)
(72) Inventor: ICHIKAWA, Hiroaki, 1920912 (JP); ONUKI, Yoshio, 1920917 (JP); ONISHI, Norio, 1970012 (JP); YAMAMOTO, Tetsuya, 3570044 (JP); MIKKAICHI, Takayasu, 1830033 (JP)
(74) Representative: von Hellfeld, Axel
(86) International application number: PCT/JP2006/311452
(87) International publication number: WO 2006/134815

(56) References cited:
- WO-A1-96/32146
- JP-A- 2000 279 418
- JP-A- 2000 516 832
- JP-A- 2003 093 393
- US-A- 5 882 600
- US-A1- 2004 068 291

## Description

### BACKGROUND OF THE INVENTION

### Field of the Invention

This invention relates to a tissue capturing device that captures biological tissue that has been suctioned and recovered from inside a patient. The present invention also relates to a treatment tool for an endoscope and an endoscope that are provided with this tissue capturing device, and that perendoscopically collect biological tissue within a patient.

### Description of Related Art

The device described below is known as a tissue capturing device that captures biological tissue that has been perendoscopically suctioned and recovered from inside a patient. Namely, a tissue capturing device in which an inner side tube that suctions and recovers biological tissue is in communication with a suction tube that is connected to a suction source, and in which a tissue trap mounting portion that is provided with a tissue confirmation window is provided in a base end portion of an endoscope treatment tool, and in which biological tissue is captured in a mesh filter as a result of a trap body which has the mesh filter being inserted into the tissue capture device is known (see, for example, Patent Document 1: Japanese Unexamined Patent Application, First Publication No. 2003-93393). In this tissue capturing device, confirmation of whether or not captured biological tissue is present as well as the condition thereof is performed visually through the tissue confirmation window.

However, in the case of a tissue capturing method such as a biopsy, because the biological tissue that is captured in the mesh filter is extremely small, in order to confirm the existence or otherwise of biological tissue, it has been necessary to approach close to the tissue confirmation window and look carefully at the interior of the tissue trap mounting portion, or to first remove the mesh filter and then make the confirmation. Moreover, because biological tissue is captured in a narrow range mesh filter as a suctioned object that is mixed together with mucus and blood and the like, a considerable time is taken up by manipulating while suctioning in order to confirm with the naked eye the presence or otherwise of biological tissue in the suctioned object, and is the cause of a considerable load falling on an operator of the tissue capturing device.

### SUMMARY OF THE INVENTION

This invention was conceived in view of the above described circumstances and it is an object thereof to provide a tissue capturing device that makes it possible to more easily confirm the presence or otherwise of tiny amounts of biological tissue.

In order to solve the above described problems, this invention has the following structure.

Namely, the present invention is a tissue capturing device that includes: a container that has an intake aperture that is connected to a fluid intake tube that transports biological tissue that has been collected by a treatment portion of a treatment tool for an endoscope and has been mixed with a liquid, and an outlet aperture that is connected to a fluid outlet tube that discharges the fluid that has been separated from the biological tissue; a filter member that forms a screen between the intake aperture and the outlet aperture inside the container, and in which tissue capturing surfaces that are able to capture the biological tissue are formed on the intake aperture side, and in which a plurality of micro through holes that are in communication with the outlet aperture side and through which only the liquid is able to pass are formed in the tissue capturing surfaces; and a lens member that is provided at a position inside the container where makes it possible to observe an enlargement of the tissue capturing surfaces.

According to the tissue capturing device of this invention, biological tissue that has been collected by a treatment portion of an endoscope treatment tool is transported to a tissue capturing device by a fluid intake tube, and is captured on a tissue capturing surface inside the tissue capturing device. A liquid that includes fluids such as blood and mucus that has been collected mixed together with the biological tissue is discharged to a fluid outlet tube through micro through holes. With the biological tissue and liquid separated in this manner, it is possible to immediately enlarge and observe the biological tissue that has been captured on the tissue capturing surface using the lens member. Because of this, it is possible to reliably confirm the presence or otherwise as well as the condition of the biological tissue.

In the present invention, it is also possible for the container to be a substantially circular cylinder-shaped member that has the intake aperture in a top portion thereof and has the outlet aperture in a bottom portion thereof, and for the filter member to be a substantially circular member that corresponds to the shape of the container, and for a plurality of capture spaces to be formed by a plurality of partition walls that are provided on the intake aperture side inside the container extending radially outwards from a center axis of the filter member, and for one of the tissue capturing surfaces to be formed in each one of the capture spaces, and for the intake aperture to be able to correspond selectively to any one of the capture spaces.

According to the tissue capturing device of this invention, a plurality of tissue capturing surfaces are formed in a filter member that is provided inside a container, and the respective tissue capturing surfaces are partitioned by partition walls from adjacent tissue capturing surfaces. Because of this, if one of the tissue capturing surfaces is placed at a position corresponding to the intake aperture to which the biological tissue is being transported and, after capture has been confirmed via the lens member, the filter member is then pivoted into the interior of the container, then it becomes possible to sequentially confirm the capture of new biological tissue. Moreover, because the plurality of tissue capturing surfaces which are adjacent to each other are separated by partitioning walls, there is no possibility of captured biological tissues becoming intermingled.

In the present invention, it is also possible for a plurality of the lens members to be provided so as to correspond to a plurality of provided tissue capturing surfaces.

According to the tissue capturing device of this invention, it is possible to confirm at an appropriate time the state of capture on a particular tissue capturing surface while this tissue capturing surface is capturing the biological tissue. Furthermore, because lens members are also provided to correspond to the other tissue capturing surfaces, depending on the condition at a particular time, it is possible to complete the capturing and also enlarge and observe at an appropriate time the condition of whichever biological tissue is to be observed.

In the present invention, it is also possible for the container to be provided with a case in which a removal aperture for removing the filter member is provided, and with a lid that can be removably fitted onto the removal aperture.

According to the tissue capturing device of this invention, because a removal aperture for removing the filter member is provided in the container, after the capturing of the biological tissue has ended, it is possible to separate the filter member from the container while it still holds the captured biological tissue.

In the present invention, it is also possible for a pair of engaging portions that are able to be engaged with each other to be provided in the lid and the filter member, and in a state in which the pair of engaging portions are engaged with each other, for the lid and the filter member to be able to be removed from the case of the container.

According to the tissue capturing device of this invention, it is possible to remove as a single unit both the filter member while it still holds the captured biological tissue and the lid from the tissue capturing device. Because of this, storage of the filter member after capturing is simplified and, in the same way as is described above, it is possible to confirm biological tissue by observing an enlargement thereof even when the filter member is being stored.

In the present invention, it is also possible for the lens member to be provided at a position facing the tissue capturing surfaces.

According to the tissue capturing device of this invention, as a result of the lens member being provided at a position facing the tissue capturing surface where biological tissue has been captured, it becomes even easier to confirm biological tissue by observing an enlargement thereof.

In the present invention, it is also possible for a bowl-shaped recessed portion that is large enough to capture the biological tissue to be formed on the intake aperture side of the filter member, and for the tissue capturing surface to be provided in a bottom surface of the recessed portion.

According to the tissue capturing device of this invention, because a bowl-shaped recessed portion is formed, biological tissue is accumulated more efficiently on the tissue capturing surface of the bottom surface. As a result, it becomes even easier to confirm biological tissue by observing an enlargement thereof.

In the present invention, it is also possible for there to be provided an illumination member that illuminates the tissue capturing surface.

According to the tissue capturing device of this invention, because it is possible to observe an enlargement of captured biological tissue, in a state in which the captured biological tissue is being illuminated by an illumination member, it becomes even easier to confirm biological tissue.

The present invention is a treatment tool for an endoscope that includes: the tissue capturing device; the fluid intake tube that has a first distal end portion that is able to suction a target position inside a patient, and a first base end portion that is able to be connected to the intake aperture of the tissue capturing device; and the fluid outlet tube that has a second distal end portion that is able to be connected to the outlet aperture of the tissue capturing device, and a second base end portion that is connected to a suction source.

In the treatment tool for an endoscope of this invention, because a tissue capturing device that makes possible enlargement and observation using a lens member is mounted thereon, it is possible to immediately enlarge and observe the condition of biological tissue that has been captured by the treatment portion of an endoscope treatment tool.

The present invention is an endoscope that includes: the tissue capturing device; the fluid intake tube that has a first distal end portion that is able to suction a target position inside a patient, and a first base end portion that is able to be connected to the intake aperture of the tissue capturing device; and the fluid outlet tube that has a second distal end portion that is able to be connected to the outlet aperture of the tissue capturing device, and a second base end portion that is connected to a suction source.

In the endoscope of this invention, because a tissue capturing device that makes possible enlargement and observation using a lens member is mounted thereon, it is possible to immediately enlarge and observe the condition of biological tissue that has been captured by the treatment portion of an endoscope treatment tool.

According to the present invention, it is possible to use a lens member to enlarge and observe biological tissue that is on a tissue capturing surface of a filter member and has been separated out from liquids such as body fluids. As a result, it is no longer necessary to actually carry the case to an observer and observe the biological tissue from up close, or to first remove the filter and then observe the biological tissue from up close. Because of this, it is possible to reliably confirm the presence or otherwise of biological tissue as well as the condition thereof, and there is no need to repeat the operation to capture biological tissue because of an unsatisfactory confirmation. Namely, it is possible to reduce work time needed for the capturing operation, and also lighten the load on the operator of the tissue capturing device. Consequently, the load on a patient who is being examined is also lightened.

### BRIEF DESCRIPTION OF THE DRAWINGS

FIG. 1 is an overall view showing a treatment tool for an endoscope of a first embodiment of this invention.
FIG. 2 is an enlarged view showing a treatment portion of the treatment tool for an endoscope of the first embodiment of this invention.
FIG. 3 is a perspective view showing the tissue capturing device of the first embodiment of this invention.
FIG. 4 is a cross-sectional view showing the tissue capturing device of the first embodiment of this invention.
FIG. 5 is a cross-sectional view showing the tissue capturing device of the first embodiment of this invention.
FIG. 6 is a perspective view showing a filter member of the tissue capturing device of the first embodiment of this invention.
FIG. 7 is a perspective view showing a filter member of the tissue capturing device of the first embodiment of this invention.
FIG. 8 is a perspective view showing a lid of the tissue capturing device of the first embodiment of this invention.
FIG. 9 is a perspective view showing a case of the tissue capturing device of the first embodiment of this invention.
FIG. 10 is a cross-sectional view showing a specimen case of the tissue capturing device of the first embodiment of this invention.
FIG 11 is a perspective view showing a case of the tissue capturing device of a second embodiment of this invention.
FIG. 12 is a cross-sectional view showing the tissue capturing device of the second embodiment of this invention.
FIG. 13 is a perspective view showing the tissue capturing device of a third embodiment of this invention.
FIG. 14 is a perspective view showing a filter member of the tissue capturing device of the third embodiment of this invention.
FIG 15 is a cross-sectional view showing the tissue capturing device of the third embodiment of this invention.
FIG. 16 is a perspective view showing the tissue capturing device of a fourth embodiment of this invention.
FIG. 17 is a cross-sectional view showing the tissue capturing device of the fourth embodiment of this invention.
FIG. 18 is a perspective view showing the tissue capturing device of a fifth embodiment of this invention.
FIG. 19 is a cross-sectional view showing the tissue capturing device of the fifth embodiment of this invention.
FIG. 20 is an overall view showing an endoscope of the sixth embodiment of this invention.
FIG. 21 is an overall view showing an endoscope of a variant example of the sixth embodiment of this invention.

### DETAILED DESCRIPTION OF THE INVENTION

### (First embodiment)

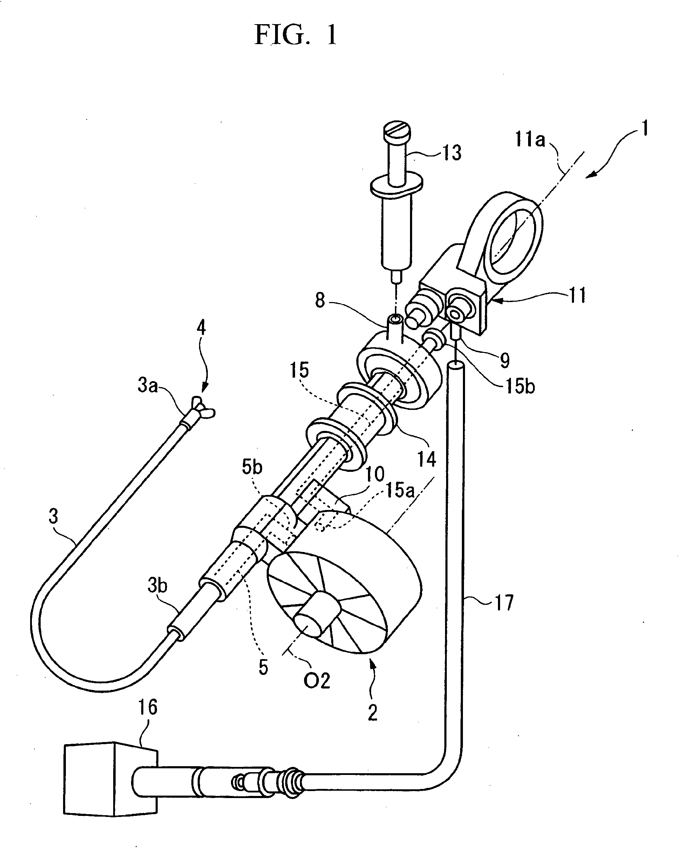

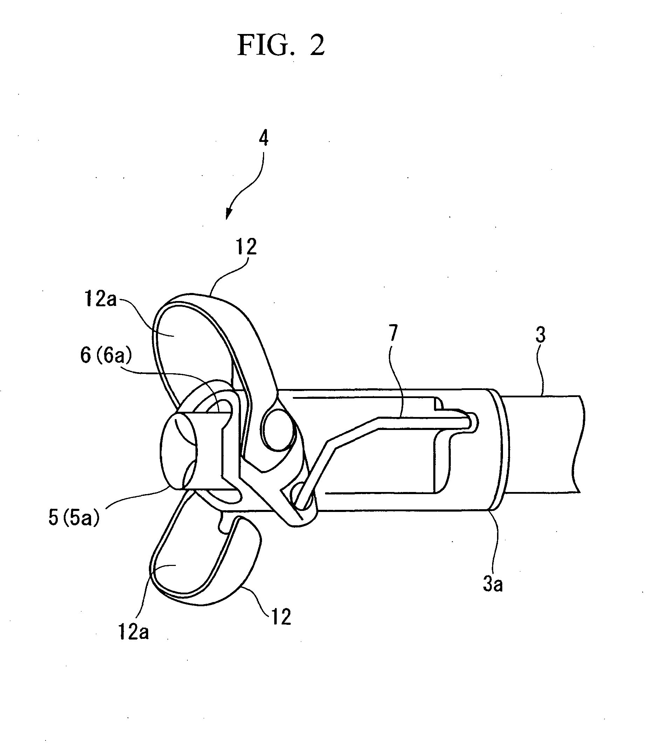

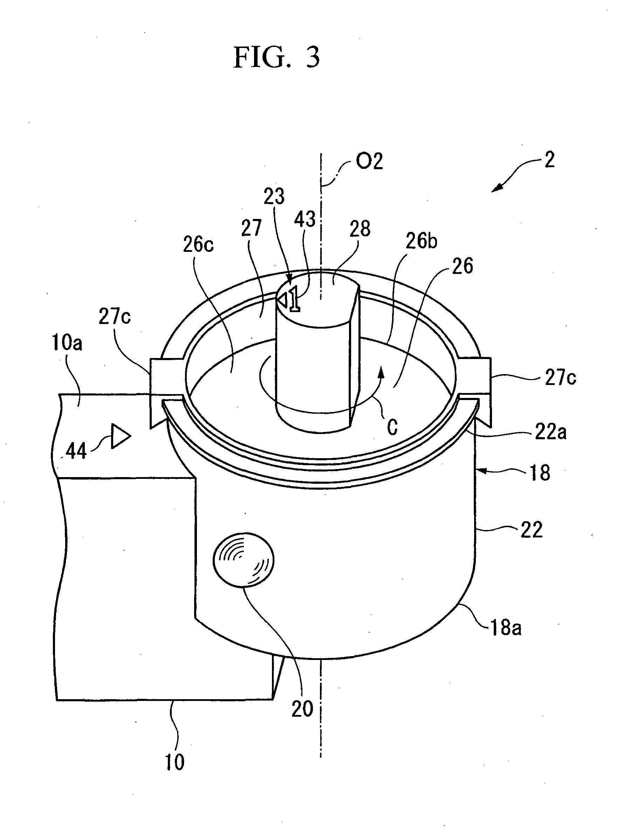

FIG. 1 through 10 show a first embodiment of this invention. FIG. 1 is an overall view of a treatment tool 1 for an endoscope in which a tissue capturing device 2 is provided, while FIG. 2 is an enlarged view of a treatment portion 4 of the treatment tool 1 for an endoscope. In addition, FIG 3 is an enlarged view of the tissue capturing tool 2 that is fitted onto the treatment tool 1 for an endoscope.

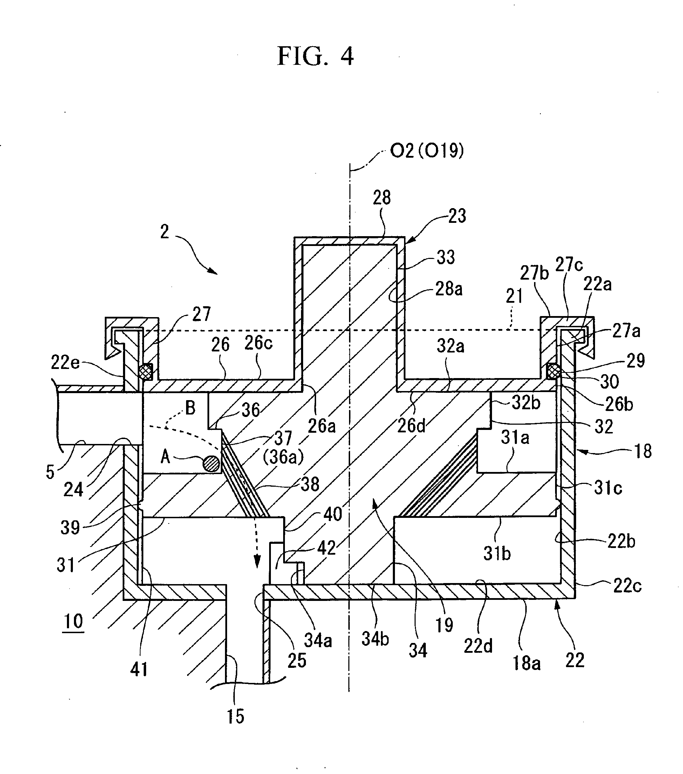

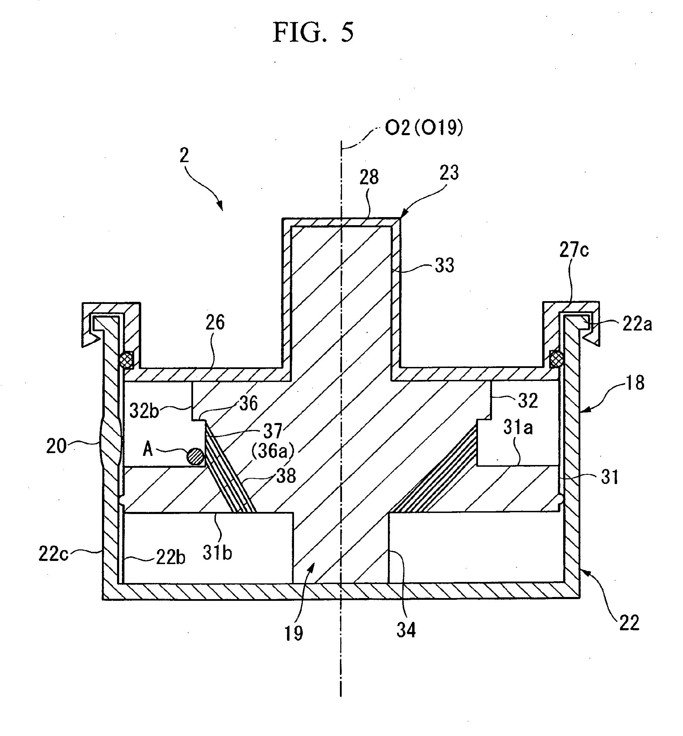

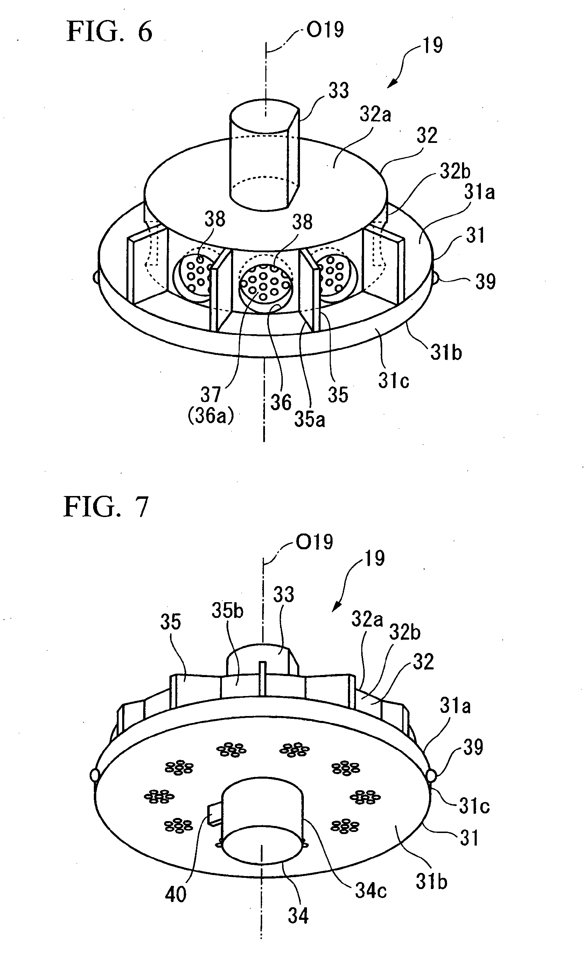

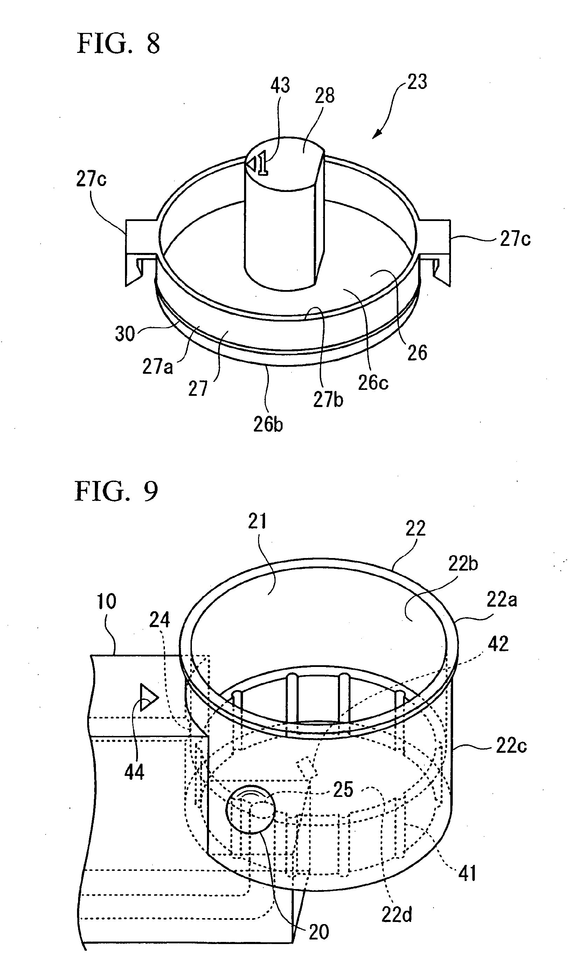

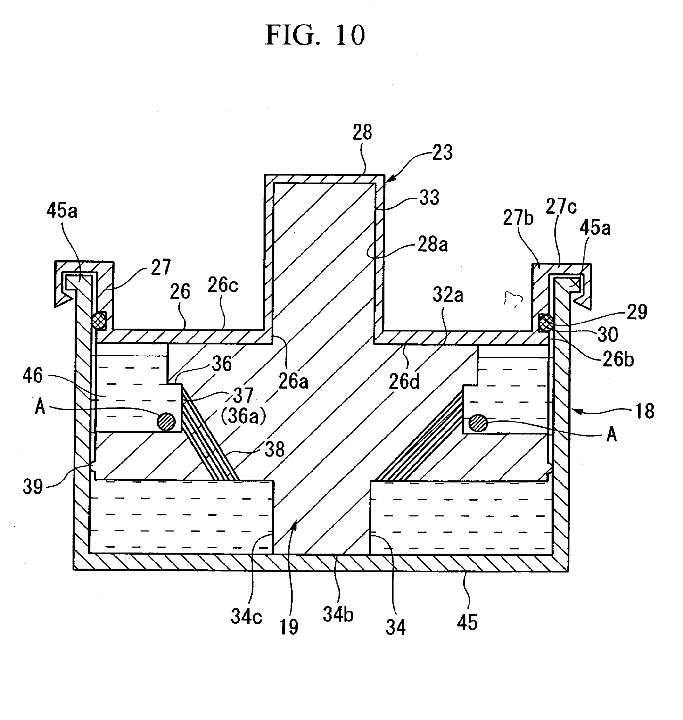

FIG. 4 is a cross-sectional view of the tissue capturing device 2 at the position of an intake aperture 24, while FIG. 5 is a cross-sectional view of the tissue capturing device 2 at the position of a lens member 20. Furthermore, FIG. 6 and FIG. 7 are perspective views of a filter member 19 of the tissue capturing device 2. FIG. 8 is a perspective view of a lid 23 of the tissue capturing device 2, while FIG. 9 is a perspective view of a case 22 of the tissue capturing device 2. FIG. 10 is a cross-sectional view of a specimen case 45 of biological tissue A that has been captured by the tissue capturing device 2.

As is shown in FIG. 1, the treatment tool 1 for an endoscope of this invention is formed by the tissue capturing device 2, a flexible tube 3 that is inserted into a patient and has flexibility, the treatment portion 4 that is provided on a distal end portion 3a of the flexible tube 3, a fluid intake tube 5 that is fitted onto the flexible tube 3, a water supply tube 6 and operating wire 7, and an operating portion 11 that is connected to a base end portion 3b of the flexible tube 3 and in which are provided a water supply port 8, a suction tube connection aperture 9, and a device mounting portion 10 to which the tissue capturing device 2 is able to be connected. The device mounting portion 10 is mounted such that a center axis 02 of the tissue capturing device 2 is parallel with an operation axis 11a of the operating portion 11.

As is shown in FIG. 2, the treatment portion 4 is fixed to the distal end portion 3a of the flexible tube 3 and has a pair of jaws 12 which are both fastened on the same shaft. The pair of jaws 12 are able to be opened and closed as a result of the operating wire 7 moving in conjunction with a slider 14. A distal end portion 5a of the fluid intake tube 5 that is fitted onto the flexible tube 3 and a distal end portion 6a of the water supply tube 6 are exposed between the pair of jaws 12. As is shown in FIG. 1, when the base end portion 5b of the fluid intake tube 5 is connected to the device mounting portion 10, and the tissue capturing device 2 is connected to the device mounting portion 10, the base end portion 5b of the fluid intake tube 5 is connected to the tissue capturing device 2. In addition, a base end portion of the water supply tube 6 is connected to the water supply portion 8. A water supply source 13 is also connected to the water supply port 8. For example, a syringe or a water supply pump is provided as a water supply device. A fluid outlet tube 15 is fitted inside the operating portion 11. When a distal end portion 15a of the fluid outlet tube 15 is connected to the device mounting portion 10, and the tissue capturing device 2 is connected to the device mounting portion 10, the distal end portion 15a of the fluid outlet tube 15 is connected to the tissue capturing device 2. In addition, a base end portion 15b of the fluid outlet tube 15 is connected to the suction tube connection aperture 9 of the operating portion 11, and a suction tube 17 that is connected to a suction source 16 is able to be connected to the suction tube connection aperture 9.

As is shown in FIG. 3 and FIG. 4, the tissue capturing device 2 is formed by a container 18, a filter member 19 that is able to be housed inside the container 18, and a lens member 20. The container 18 is a substantially circular cylinder-shaped member, and is provided with a case 22 and a lid 23. A bottom portion 18a of the case 22 is closed, while a top portion 18b of the case 22 is open so as to form an extraction aperture 21 for the filter member 19. The lid 23 closes the extraction aperture 21. The case 22 and the lid 23 are formed from a transparent plastic such as, for example, polycarbonate or polysulfone. The filter member 19 is provided on the inner side of the container 18, and the filter member 19 is sandwiched from above and below by the case 22 and the lid 23. Moreover, as is shown in FIG. 4, an intake aperture 24 that is connected to the base end portion 5b of the fluid intake tube 5 is formed on a top portion of a side surface 22e of the case 22, and an outlet aperture 25 to which the distal end portion 15a of the fluid outlet tube 15 is connected is formed in the bottom portion 18a of the case 22. In addition, a lid engaging portion 22a is provided on an outer circumferential edge of the top of the case 22.

As is shown in FIG. 3, FIG. 4, and FIG. 8, the lid 23 is a substantially circular plate-shaped member whose outer diameter is set so as to be slightly smaller than the inner diameter of the case 22. The lid 23 is formed by a circular disk portion 26 that has a through hole 26a provided in the center thereof, a circumferential vertical wall portion 27 that protrudes upwards from an outer circumferential edge 26b of the circular disk portion 26, and a knob portion 28 that protrudes upwards coaxially with a top surface 26c of the circular disk portion 26. A hollow portion 28a is formed inside the knob portion 28, and the hollow portion 28a is connected to the through hole 26a in the circular disk portion 26. As is shown in FIG. 3 and FIG. 8, a planar cross-section of the knob portion 28 and the through hole 26a of the circular disk portion 26 is a non-circular shape. More specifically, these are formed having a D-shape. Moreover, as is shown in FIG. 4 and FIG. 8, an O-ring mounting groove 29 is formed in an outer circumferential portion 27a of the circumferential vertical wall portion 27, and an O-ring 30 is fitted therein. When the lid 23 is assembled in the case 22 using the circumferential vertical wall portion 27 and the O-ring 30 of the lid 23, the extraction aperture 21 can be kept air-tight. Case engaging portions 27c are formed on the top end portion 27b of the circumferential vertical wall portion 27, and when assembling the case 22 and the lid 23, the case 22 and the lid 23 can be formed into a single unit by engaging the case engaging portions 27c in the lid engaging portion 22a.

As is shown in FIG 4, FIG 6, and FIG. 7, the filter member 19 is formed by a substantially circular plate-shaped partitioning portion 31, a circular column portion 32 that is formed substantially in the shape of a circular column and is provided coaxially with a top surface 31a of the partitioning portion 31, an engaging portion 33 that is provided coaxially with a top surface 32a of the circular column portion 32, and a shaft portion 34 that is formed substantially in a circular column shape and is provided coaxially with a bottom surface 31b of the partitioning portion 31. The outer diameter of the partitioning portion 31 is set such that, when the filter member 19 is inserted into the case 22, the gap between an outer circumferential portion 31 c of the partitioning portion 31 and an inner circumferential portion 22b of the case 22 is a size that prevents biological tissue A which has been recovered from a patient by the treatment portion 4 passing therethrough. The outer diameter of the circular column portion 32 is set smaller than the outer diameter of the partitioning portion 31. In addition, the planar cross-sectional configuration of the engaging portion 33 has a D-shape that corresponds to the shape of the hollow portion 28a of the knob portion 28 and the through hole 26a of the circular disk portion 26, and is set to a size that allows the engaging portion 33 to be engaged in the hollow portion 28a of the knob portion 28. Furthermore, the outer diameter of the shaft portion 34 is set smaller than the outer diameter of the partitioning portion 31. The filter member 19 is formed from plastic, and may be either a transparent material or a non-transparent material, however, it is preferably a blue color.

As is shown in FIG. 6 and FIG. 7, a plurality of partition walls 35 that protrude radially from a center axis 019 of the filter member 19 as far as the outer circumferential portion 31 c of the partitioning portion 31 are placed at equidistant intervals on a circumferential surface portion 32b of the circular column portion 32. A bottom edge 35a of each partition wall 35 is joined to the top surface 31a of the partitioning portion 31, and the height of the partition walls 35 is the same as the height of the circular column portion 32. In respective capture spaces 35b that are partitioned by the plurality of partition walls 35, recessed portions 36 that are each of a size that makes it possible to capture biological tissue A which has been recovered from a patient by the treatment portion 4 are formed in the circumferential surface portion 32b of the circular column 32. A tissue capturing surface 37 is formed on a bottom surface 36a of each recessed portion 36. A plurality of micro through holes 38 that penetrate as far as a bottom surface 31b of the partitioning portion 31 are formed in the respective tissue capturing surfaces 37 of each recessed portion 36. The size of the micro through holes 38 is set so as to prevent biological tissue A which has been recovered from a patient by the treatment portion 4 from passing through, while allowing fluids such as blood and mucus and the like that were recovered together with the biological tissue A to pass through. In addition, at least one positioning bump 39 that normally has a protruding shape, but that can be easily deformed by external pressure is formed on the outer circumferential portion 31c of the partitioning portion 31. Furthermore, a stopper rib 40 protrudes in a radial direction from a circumferential wall portion 34a of the shaft portion 34.

Moreover, as is shown in FIG. 4, FIG. 5, and FIG. 9, a plurality of positioning recessed portions 41 which are grooves with which the positioning bump 39 of the filter member 19 can be engaged are formed in the inner circumferential portion 22b of the case 22 of the container 18. The plurality of positioning recessed portions 41 are placed at the same spacing in the circumferential direction as the placement spacing of the recessed portions 36 of the filter member 19. When the filter member 19 is inserted into the interior of the case 22, and the positioning bump 39 of the filter member 19 is engaged in one of the positioning recessed portions 41 of the case 22, then the tissue capturing surface 37 of the corresponding recessed portion 36 faces the intake aperture 24 that is formed in the case 22. In addition, the lens member 20 is mounted in the outer circumferential portion 22c of the case 22 so as to extend from the outer circumferential portion 22c to the inner circumferential portion 22b. The lens member 20 is formed, for example, from plastic or glass or the like. The mounting position of the lens member 20 is a position facing another tissue capturing surface 37 which is adjacent to the tissue capturing surface 37 facing the intake aperture 24 when the filter member 19 is inserted into the case 22. Furthermore, a stopper protrusion 42 protrudes from a bottom surface 22d of the case 22. When the positioning bump 39 of the filter member 19 is engaged with one of the positioning recessed portions 41 of the case 22, the stopper protrusion 42 is positioned so as to abut against a stopper rib 40 such that the filter member 19 does not pivot inside the case 22 in the opposite direction from a pivot direction C that matches a circumferential direction moving from the intake aperture 24 towards mounting position of the lens member 20.

By employing the above described structure, as is shown in FIG. 4, the tissue capturing device 2 is assembled in a state in which, inside the case 22, the bottom surface 22d of the case 22 is abutting against the bottom surface 34b of the shaft portion 34 of the filter member 19, the bottom surface 26d of the circular disk portion 26 of the lid 23 is abutting against the top surface 32a of the circular column portion 32, and the hollow portion 28a of the knob portion 28 of the lid 23 is abutting against the engaging portion 33 of the filter member 19. Because of this, the lid 23 and the filter member 19 are able to pivot around the center axis 02 inside the case 22 by means of the shaft portion 34 of the filter member 19 and the case engaging portion 27c of the lid 23 and lid engaging portion 22a of the case 22. Moreover, the engaging force between the case engaging portion 27c of the lid 23 and the lid engaging portion 22a of the case 22 is set smaller than the engaging force between the knob portion 28 of the lid 23 and the engaging portion 33 of the filter member 19. Because of this, if the knob portion 28 of the lid 23 is gripped and the knob portion 28 is pulled out from the case 22, then the lid 23 and the filter member 19 can be removed as a single unit. In addition, corresponding positioning markings 43 and 44 are provided on the top surface 28b of the knob portion 28 of the lid 23 and the top surface 10a of the device mounting portion 10. When the tissue capturing device 2 is being mounted on the device mounting portion 10, the knob portion 28 of the lid 23 is pivoted and adjusted such that the positioning markings 43 and 44 are positioned opposite each other. As a result, in a state in which the positioning bump 39 of the filter member 19 and a positioning recessed portion 41 are engaged together, and in which the stopper rib 40 is abutting against the stopper protrusion 42, the intake aperture 24 and one of the tissue capturing surfaces 37 are set so as to face each other.

Next, an operation of the treatment tool 1 for an endoscope and the tissue capturing device 2 of this invention will be described. Firstly, the flexible tube 3 of the treatment tool 1 for an endoscope shown in FIG. 1 is inserted into a patient using a channel of an endoscope (not shown). Once the treatment portion 4 has reached a target position, as is shown in FIG. 2, the slider 14 is pushed towards the distal end side so that the pair of jaws 12 is opened. While the pair of jaws 12 is open, the treatment portion 4 is brought close to the target position, and the slider 14 is then pulled towards the base end side so as to close the pair of jaws 12. As a result, the biological tissue A at the target position becomes housed inside the hollow portion 12a of the closed pair of jaws 12. Next, water from the water supply source 13 passes via the water supply port 8 through the water supply tube 6, and is discharged from the distal end portion 6a of the water supply tube 6. The collected biological tissue A together with fluids such as blood and mucus and the like are mixed with this discharged water in the hollow portion 12a of the jaws 12, so as to form a fluid mixture. In this state, suctioning by the suction source 16 is commenced. The suction source 16 is connected via the suction tube 17 to the suction tube connection aperture 9. Furthermore, the suction source 16 is connected through the suction tube connection aperture 9 via the fluid outlet tube 15 to the outlet aperture 25 of the tissue capturing device 2, and is also connected through the intake aperture 24 of the tissue capturing device 2 via the fluid intake tube 5 to the distal end portion 5a of the fluid intake tube 5 which is exposed at the hollow portion 12a of the jaws 12. Because of this, when suctioning by the suction source 16 starts, the fluid mixture including the biological tissue A is suctioned from the hollow portion 12a of the jaws 12, passes through the fluid intake tube 5, and is fed through the intake aperture 24 into the tissue capturing device 2. At this time, as is shown in FIG. 3, the positioning marking 43 on the tissue capturing device 2 and the positioning marking 44 on the device mounting portion 10 are set in positions where they face each other.

As is shown in FIG. 4, the fluid mixture which includes the biological tissue A passes through an inter-aperture suction path B inside the tissue capturing device 2. At this time, the mixture which is in the form of a liquid made up of bodily fluids and water passes via the tissue capturing surface 37 of the recessed portion 36 through the micro through holes 38 and is transported to the outlet aperture 25. Because the biological tissue A which is a solid cannot pass through the micro through holes 38 in the tissue capturing surface 37, it stays caught on the tissue capturing surface 37. Once biological tissue A that has been collected by the jaws 12 has been sufficiently transported, operations of the water supply source 13 and the suction source 16 are stopped. In this state, the knob portion 28 of the lid 23 is gripped and pivoted in the pivot direction C. Here, because the engaging portion 33 and the knob portion 28 are in a state of engagement with each other, the filter member 19 moves in conjunction with the lid 23 and is pivoted around the center axis 02 inside the case 22. At this time, the positioning bump 39 of the filter member 19 comes out from the positioning recessed portion 41 of the case 22 and is deformed, thereby enabling the filter member 19 to pivot. However, because the positioning bump 39 is only allowed to pivot in the pivot direction C by the stopper rib 40 of the shaft portion 34 of the filter member 19 and the stopper protrusion 42 of the case 22, it is not possible for the filter member 19 to pivot in the opposite direction from the pivot direction C.

By causing the knob portion 28 of the lid 23 to pivot, the tissue capturing surface 37 that is facing the intake aperture 24 and has captured the biological tissue A is moved to a position where it faces the lens member 20. The positioning bump 39 which had been pushed inside the outer circumferential portion 31c of the partitioning portion 31 is then also moved, and becomes engaged with the next positioning recessed portion 41, and the lens member 20 and the tissue capturing surface 37 become placed in mutually facing positions. The biological tissue A is then viewed frontally and in enlargement by the lens member 20, and the presence or otherwise of captured biological tissue A as well as the condition thereof can be confirmed. Accordingly, it can be said that there is an improvement in reliability and viewability. Moreover, because a new tissue capturing surface 37 that is adjacent to the confirmed tissue capturing surface 37 is placed in a position facing the intake aperture 24, it is possible at the same time to start the capturing of the next biological tissue A. At this time, because mutually adjacent tissue capturing surfaces 37 are separated from each other by the partition walls 35, there is no possibility that captured biological tissues A will become intermingled.

By repeating this operation in sequence, it is possible to capture the same number of biological tissue A samples as there are tissue capturing surfaces 37 formed in the filter member 19. If the knob portion 28 of the lid 23 is rotated after biological tissue A has been captured by the last tissue capturing surface 37, then prior to the tissue capturing surface 37 which captured the first biological tissue A that is next in the sequence being pivoted to the position facing the intake aperture 24, the stopper rib 40 of the shaft portion 34 of the filter member 19 once again abuts against the stopper protrusion 42 of the case 22. Because of this, notification can be given to the operator that the capturing of biological tissue A by all the tissue capturing surfaces 37 has ended, and any duplicated taking of the biological tissue A can be prevented.

As a result of the above tasks being performed, while the filter member 19 which has completed the capturing of the biological tissue A is in a state of being engaged with the lid 23, by disengaging the case engaging portions 27c of the circumferential vertical wall portion 27 of the lid 23 from the lid engaging portion 22a of the case 22, the filter member 19 can be separated from the case 22. Note that if biological tissue A that has been captured by the tissue capturing device 2 becomes dried out, it becomes autolyzed and cannot be used in a pathological examination. Because of this, it is necessary to preserve the biological tissue A by immersing it in a tissue preserving solution. As is shown in FIG. 10, a specimen case 45 is prepared having lid engaging portions 45a and having the same shape as the previously prepared case 22. The interior of the specimen case 45 is filled with tissue preserving solution 46, and the unit that is formed by the integrated lid 23 and filter member 19 is placed inside the specimen case 45. As a result, captured biological tissue A can be kept secure. In this method, there is no need to transfer the captured biological tissue A and it can be stored without being handled. Moreover, by preparing another lid 23 and filter member 19 and installing them anew in the tissue capturing device 2, it is possible to capture biological tissue A that is additionally acquired from a patient.

An operation of this embodiment has been described above, however, in the tissue capturing device 2 of this embodiment, by providing the lens member 20 it is possible to enlarge and accurately confirm captured biological tissue A. As a result, because it is no longer necessary to carry the case 22 to an observer and observe the biological tissue A from up close, or to first remove the filter and then observe the biological tissue A from up close, it is possible to reduce work time and also lighten the load on the operator of the tissue capturing device. Moreover, in the case of the tissue capturing device 2 of this embodiment, because the place where the fluid mixture containing the biological tissue A flows into the tissue capturing device 2 can be separated by the partition walls 35 from the place where the biological tissue A is observed using the lens member 20, there is no contamination of the lens member 20 by the fluid mixture which might result in it becoming difficult to make an observation using the lens member 20, so that a convenient and also hygienic tissue capturing device can be realized.

Note that although the lens member 20 has been mounted in the case 22, it is also possible for the case 22 and the lens member 20 to be formed as a single unit. In addition, the lid 23 and the filter member 19 are engaged with both the knob portion 28 and the engaging portion 33 each having a D-shaped planar cross sectional configuration, however, the present invention is not limited to this and it is sufficient if there is enough engaging force to prevent the two from separating when they are being pivoted or extracted.

### (Second embodiment)

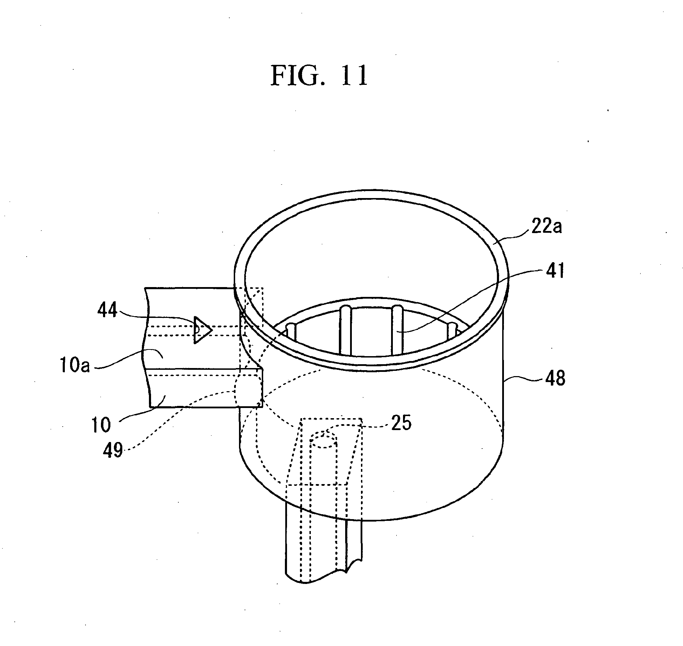

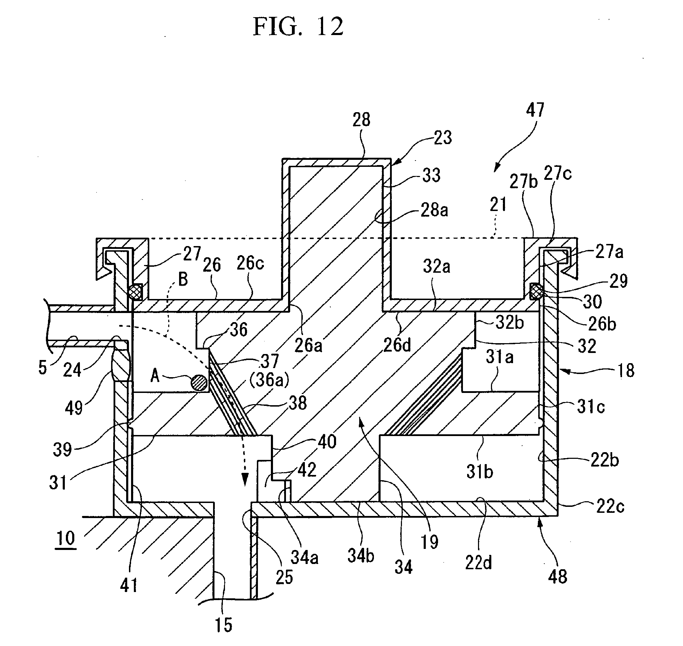

FIG. 11 and FIG. 12 show a second embodiment of this invention. In this embodiment, members that are the same as those used in the above described embodiment are given the same descriptive symbols and a description thereof is omitted. FIG. 11 is a perspective view showing a case 48 of a tissue capturing device 47 of this embodiment. FIG. 12 is a cross-sectional view showing the tissue capturing device 47 at the position of the intake aperture 24 of this embodiment.

As is shown in FIG. 11, in the tissue capturing device 47 of this embodiment, a lens member 49 is provided directly beneath the intake aperture 24. Because of this, biological tissue A that has been sent from the intake aperture 24 and captured in the tissue capturing surface 37 which is positioned opposite the intake aperture 24 can be observed in an enlarged form and confirmed at that position using the lens member 49 even during the water supply and suctioning operations. Accordingly, because it is possible to determine at an opportune time whether or not the biological tissue A has been captured in a tissue capturing surface 37, there is no occurrence of problems such as it subsequently being confirmed that biological tissue A could not be captured and the capturing task needing to be performed once again. There is neither any occurrence of problems such as a tissue capturing surface 37 becoming contaminated and that tissue capturing surface 37 becoming unfit for use.

### (Third embodiment)

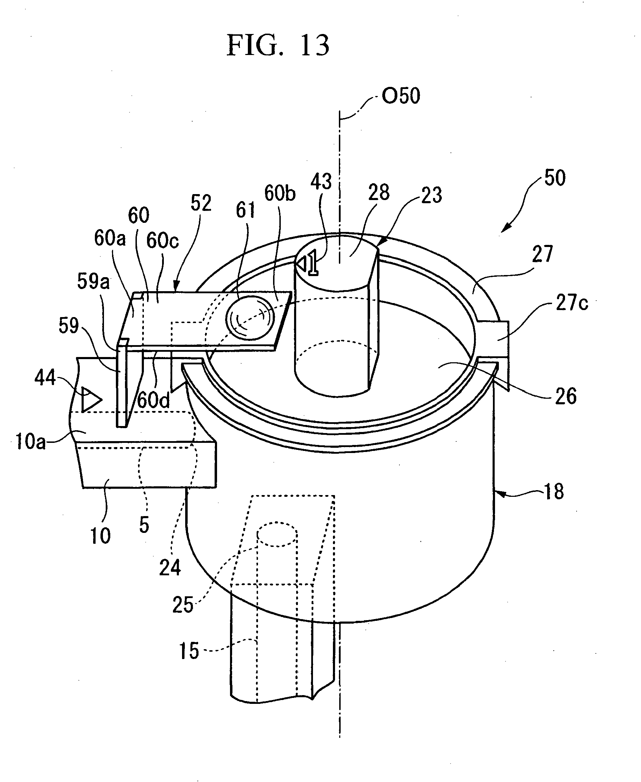

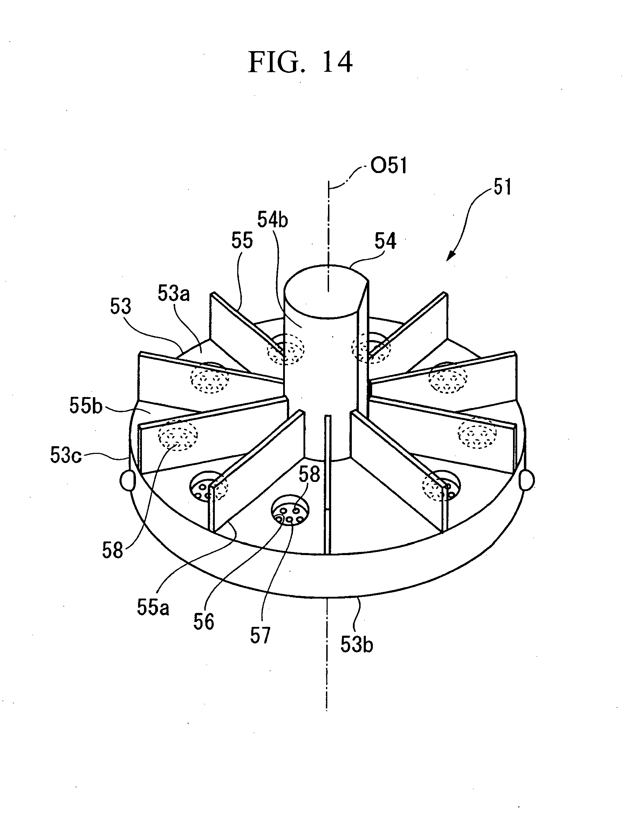

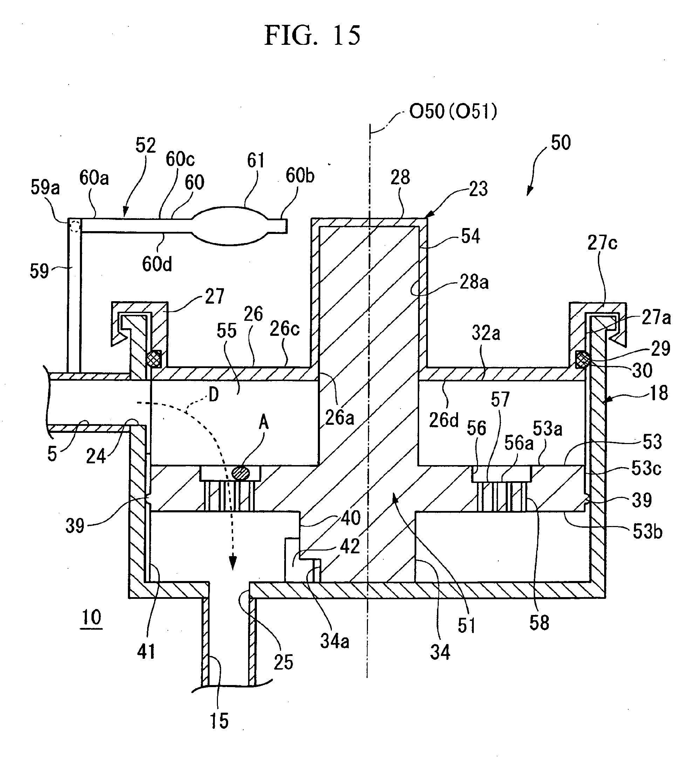

FIG. 13 through FIG 15 show a third embodiment of this invention. In this embodiment, members that are the same as those used in the above described embodiments are given the same descriptive symbols and a description thereof is omitted. FIG 13 is a perspective view showing a tissue capturing device 50 of this embodiment that is mounted on the device mounting portion 10. FIG 14 is a perspective view showing a filter member 51 inside the tissue capturing device 50. FIG. 15 is a cross-sectional view showing the tissue capturing device 50.

As is shown in FIG. 13 and FIG 15, the tissue capturing device 50 of this embodiment is formed by a container 18, a filter member 51 that is able to be housed inside the container 18, and a lens member 52. As is shown in FIG. 14 and FIG 15, the filter member 51 is formed by a substantially circular plate-shaped partitioning portion 53, an engaging portion 54 that is provided coaxially with a top surface 53a of the partitioning portion 53, and a shaft portion 34 that is formed substantially in a circular column shape and is provided coaxially with a bottom surface 53b of the partitioning portion 53.

The outer diameter of the partitioning portion 53 is set such that, when the filter member 51 is inserted into the case 22, the gap between an outer circumferential portion 53c of the partitioning portion 53 and an inner circumferential portion 22b of the case 22 is a size that prevents biological tissue A which has been recovered from a patient by the treatment portion 4 passing therethrough.

In addition, the planar cross-sectional configuration of the engaging portion 54 has a D-shape that corresponds to the shape of the hollow portion 28a of the knob portion 28 and the through hole 26a of the circular disk portion 26, and is set to a size that allows the engaging portion 54 to be engaged in the hollow portion 28a of the knob portion 28. Furthermore, the outer diameter of the shaft portion 34 is set smaller than the outer diameter of the partitioning portion 53. The filter member 51 is formed from plastic, and may be either a transparent material or a non-transparent material, however, it is preferably a blue color.

As is shown in FIG. 14, a plurality of partition walls 55 that protrude radially from a center axis O51 of the filter member 51 as far as the outer circumferential portion 53c of the partitioning portion 53 are placed at equidistant intervals on a circumferential surface portion 54b of the engaging portion 54. A bottom end 55a of each partition wall 55 is joined to the top surface 53a of the partitioning portion 53. Moreover, as is shown in FIG. 15, the height of the partition walls 55 is such that, when the engaging portion 54 of the filter member 51 is engaged with the hollow portion 28a of the knob portion 28 of the lid 23, it is held between the bottom surface 26d of the circular disk portion 26 of the lid 23 and the top surface 53a of the partitioning portion 53. In respective capture spaces 55b that are partitioned by the plurality of partition walls 55, recessed portions 56 that are each of a size that enables them to capture biological tissue A which has been recovered from a patient by the treatment portion 4 are formed on the top surface 53a of the partitioning portion 53. A tissue capturing surface 57 is formed on a bottom surface 56a of each recessed portion 56. A plurality of micro through holes 58 that penetrate as far as a bottom surface 53b of the partitioning portion 53 are formed in the respective tissue capturing surfaces 57 of each recessed portion 56. The size of the micro through holes 58 is set so as to prevent biological tissue A which has been recovered from a patient by the treatment portion 4 from passing through, while allowing fluids such as blood and mucus and the like that were recovered together with the biological tissue A to pass through. In addition, positioning bumps 39 are formed on the outer circumferential portion 53c of the partitioning portion 53, and positioning recessed portions 41 that correspond to the positioning bumps 39 are formed in the inner circumferential portion 22b of the case 22. When the positioning bumps 39 of the filter member 51 are engaged in one of the positioning recessed portions 41 of the case 22, the tissue capturing surface 57 of the corresponding recessed portion 56 is placed on a straight line that connects the intake aperture 24 and the center axis O50 of the tissue capturing device 50.

As is shown in FIG. 13 and FIG 15, the lens member 52 is formed by a stay 59, a plate 60, and a lens portion 61. The stay 59 is a substantially plate-shaped member and protrudes vertically upward from the top surface 10a of the device mounting portion 10. The plate 60 is also a substantially plate-shaped member and is able to freely pivot upwards at one end portion 60a thereof. The plate 60 is fixed by a shaft to a top end portion 59a of the stay 59 such that another end portion 60b of the plate 60 faces towards the center axis 050 of the tissue capturing device 50. The lens member 61 is provided so as to extend from a top surface 60c of the plate 60 to a bottom surface 60d thereof, and is formed from plastic or glass. Note that the device mounting portion 10 and the stay 5 may be removably fixed together, or may be permanently fixed together.

As is shown in FIG 15, the fluid mixture transported from the intake aperture 24 which includes the biological tissue A passes through an inter-aperture suction path D inside the tissue capturing device 50. At this time, the mixture which is in the form of a liquid of bodily fluids and water passes via the tissue capturing surface 57 of the recessed portion 56 through the micro through holes 58 and is transported to the outlet aperture 25. Because the biological tissue A which is in the form of a solid cannot pass through the micro through holes 58 in the tissue capturing surface 57, it stays caught on the tissue capturing surface 57. The presence or otherwise of biological tissue A captured by the tissue capturing surface 57 can be confirmed by observing an enlargement of the biological tissue A using the lens portion 61 that is positioned above the tissue capturing surface 57. At this time, an enlargement of the biological tissue A is observed through the circular disk portion 26 of the lid 23 and, because the lid 23 is formed from a transparent material, it does not block this observation. Once the capturing of the biological tissue A is completed, the knob portion 28a of the lid 23 is pivoted, and the capturing of biological tissue A by a sequential plurality of tissue capturing surfaces 57 is performed. Once all the capturing has been completed, the plate 60 of the lens member 52 is pivoted upwards so that the plate 60 does not obstruct the lid 23. After this, the lid 23 and the filter member 51 are removed from the case 22 while still in a state of engagement with each other, and the capturing task is completed. In the tissue capturing device 50 of this embodiment, in the same way as in the tissue capturing device 47 of the second embodiment, it is possible to confirm at an opportune time the presence or otherwise of the biological tissue A while the biological tissue A is being captured using the tissue capturing surfaces 57. Moreover, because the lens member 52 is provided outside the container 18, there is no contamination of the lens member 52 by a fluid mixture that includes the collected biological tissue A. Accordingly, an enlarged observation becomes even easier.

### (Fourth embodiment)

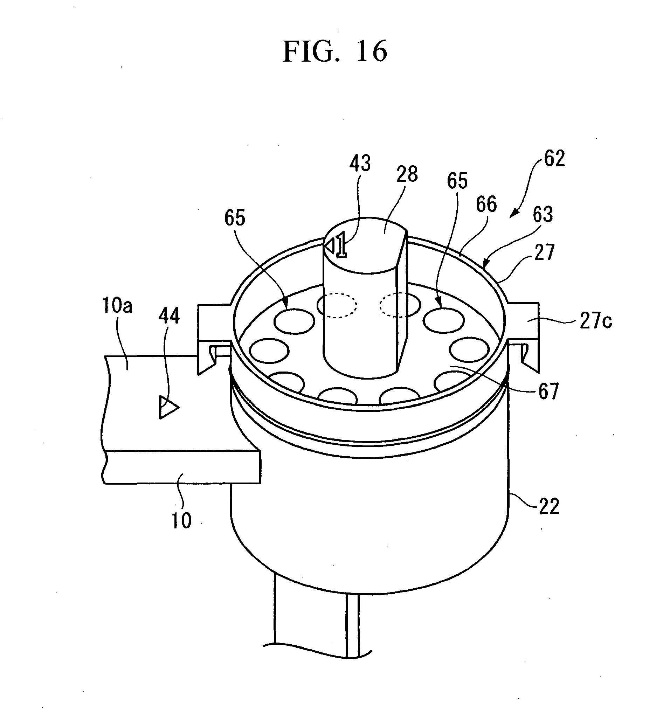

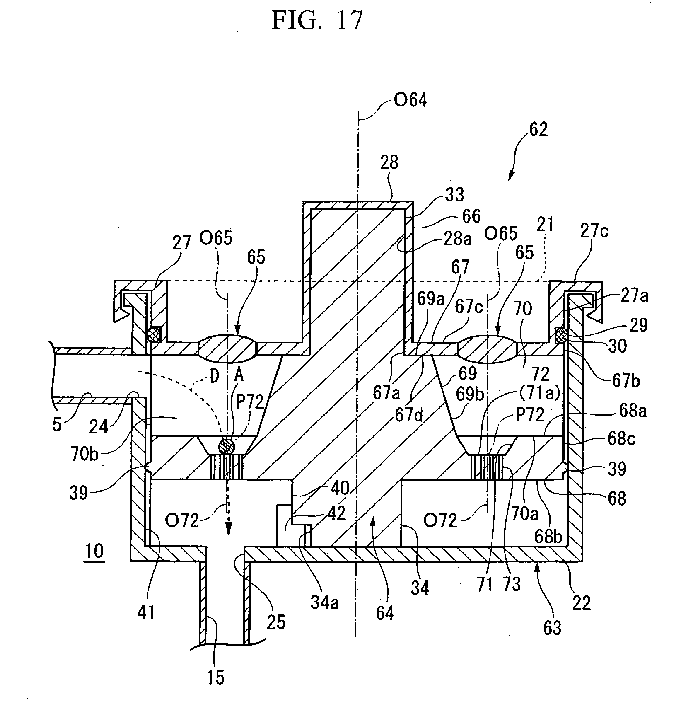

FIG. 16 and FIG. 17 show a fourth embodiment of this invention. In this embodiment, members that are the same as those used in the above described embodiments are given the same descriptive symbols and a description thereof is omitted. FIG. 16 is a perspective view showing a tissue capturing device 62 of this embodiment that is mounted on the device mounting portion 10. FIG. 17 is a cross-sectional view showing the tissue capturing device 62 of this embodiment.

As is shown in FIG 17, the tissue capturing device 62 of this embodiment is formed by a container 63, a filter member 64 that can be housed inside the container 63, and a plurality of lens members 65. The container 63 is provided with the case 22 and a lid 66, and is formed from a transparent plastic such as, for example, polycarbonate or polysulfone. The lid 66 is a substantially circular plate-shaped member whose outer diameter is set so as to be slightly smaller than the inner diameter of the case 22. The lid 66 is formed by a circular disk portion 67 that has a through hole 67a provided in the center thereof, a circumferential vertical wall portion 27 that protrudes upwards from an outer circumferential edge 67b of the circular disk portion 67, and a knob portion 28 that protrudes upwards coaxially with a top surface 67c of the circular disk portion 67.

The filter member 64 is formed by a substantially circular plate-shaped partitioning portion 68, a circular column portion 69 that is formed substantially in the shape of a circular column and is provided coaxially with a top surface 68a of the partitioning portion 68, an engaging portion 33 that is provided coaxially with a top surface 69a of the circular column portion 69, and a shaft portion 34 that is formed substantially in a circular column shape and is provided coaxially with a bottom surface 68b of the partitioning portion 68. The outer diameter of the partitioning portion 68 is set such that, when the filter member 64 is inserted into the case 22, the gap between an outer circumferential portion 68c of the partitioning portion 68 and an inner circumferential portion 22b of the case 22 is a size that prevents biological tissue A which has been recovered from a patient by the treatment portion 4 passing therethrough. The outer diameter of the circular column portion 69 is set smaller than the outer diameter of the partitioning portion 68, and has a tapered shape in which the outer diameter of the bottom portion is larger than that of the top portion. In addition, the planar cross-sectional configuration of the engaging portion 33 has a D-shape that corresponds to the shape of the hollow portion 28a of the knob portion 28 and the through hole 67a of the circular disk portion 67, and is set to a size that allows the engaging portion 33 to be engaged in the hollow portion 28a of the knob portion 28. Furthermore, the outer diameter of the shaft portion 34 is set smaller than the outer diameter of the partitioning portion 68. The filter member 64 is formed from plastic, and may be either a transparent material or a non-transparent material, however, it is preferably a blue color. A plurality of partition walls 70 that protrude radially from a center axis 064 of the filter member 64 as far as the outer circumferential portion 68c of the partitioning portion 68 are placed at equidistant intervals on a circumferential surface portion 69b of the circular column portion 69. A bottom edge 70a of each partition wall 70 is joined to the top surface 68a of the partitioning portion 68, and the height of the partition walls 70 is the same as the height of the circular column portion 69. In respective capture spaces 70b that are partitioned by the plurality of partition walls 70, recessed portions 71 that are each of a size that enable them to capture biological tissue A which has been recovered from a patient by the treatment portion 4 are formed in the top surface 68a of the partitioning portion 68. The recessed portions 71 are formed in a bowl shape having a small bottom surface 71 a. A tissue capturing surface 72 is formed on the bottom surface 71 a of each recessed portion 71. A plurality of micro through holes 73 that penetrate as far as a bottom surface 68b of the partitioning portion 68 are formed in the respective tissue capturing surfaces 72 of each recessed portion 71. The size of the micro through holes 73 is set so as to prevent biological tissue A which has been recovered from a patient by the treatment portion 4 from passing through, while allowing fluids such as blood and mucus and the like that were recovered together with the biological tissue A to pass through.

As is shown in FIG. 16 and FIG. 17, a plurality of lens members 65 are provided in the circular disk portion 67 of the lid 66 so as to extend from the top surface 67c to a bottom surface 67d thereof. The same number of lens members 65 are provided as the number of tissue capturing surfaces 72 at positions facing the respective tissue capturing surfaces 72 when the lid 66 and the filter member 64 are assembled together, and these are also positions where the center axis 065 of each lens member 65 matches the center axis 072 of each tissue capturing surface 72. Moreover, the lens members 65 and the tissue capturing surfaces 72 are positioned at a distance from each other whereby the focal points of the lens members 65 match center points P72 of the tissue capturing surfaces 72.

As is shown in FIG. 17, in this tissue capturing device 62, a fluid mixture containing biological tissue A which has been collected by the treatment portion 4 is transported from the intake aperture 24 through the inter-aperture suction path D. At this time, water and fluids such as blood and mucus pass through the through holes 73 and are transported via the outlet aperture 25 to the fluid discharge tube 15. The biological tissue A, which is solid, is captured on the tissue capturing surfaces 72. At this time, because the recessed portions 71 are formed in a bowl shape, and it is therefore difficult for the biological tissue A to become stuck on the side walls of the recessed portions 71 so that it collects in the center portions of the tissue capturing surfaces 72, it is easy to confirm the biological tissue A. Moreover, because the focal points of the lens members 65 match the center points P72 of the tissue capturing surfaces 72 and the biological tissue A is reliably collected therein, a wider observation can be easily made. Moreover, as is shown in FIG. 16 and FIG. 17, because the lens members 65 are provided at positions facing the respective tissue capturing surfaces 72, in the tissue capturing surfaces 72 where biological tissue A which has been transported from the intake aperture 24 has been captured, it is possible for the condition during the capturing operation to be confirmed in a frontal view and in enlargement. In addition, in the other tissue capturing surfaces 72, it is also possible to confirm the condition of biological tissue A whose capture is complete. Moreover, in the same way as in the method illustrated in the first embodiment, if a filter member 64 for which capturing has been completed is stored while still engaged with the lid 66, then because a lens member 65 is provided for each tissue capturing surface, even after preservation, it is possible to easily confirm the condition of the biological tissue A that is captured in the respective tissue capturing surfaces 72.

### (Fifth embodiment)

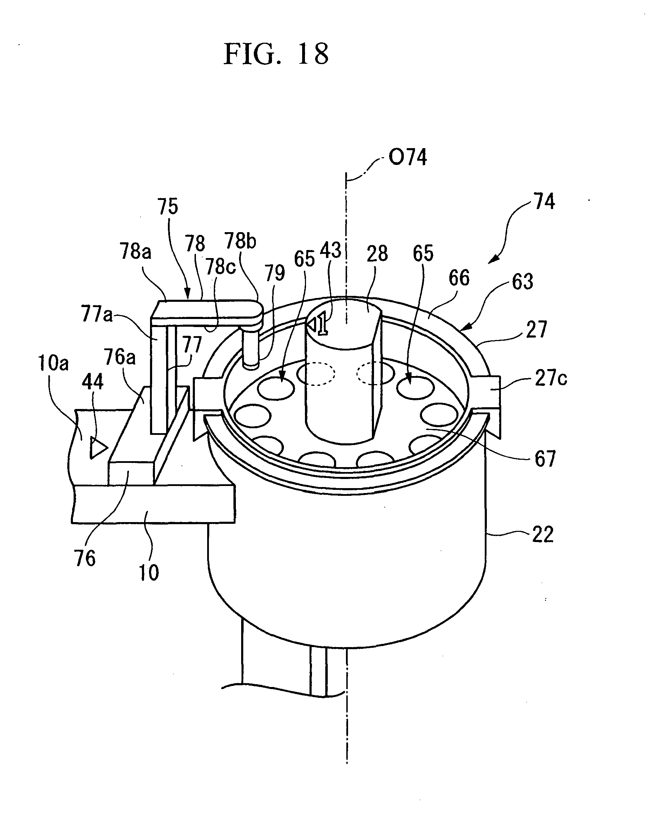

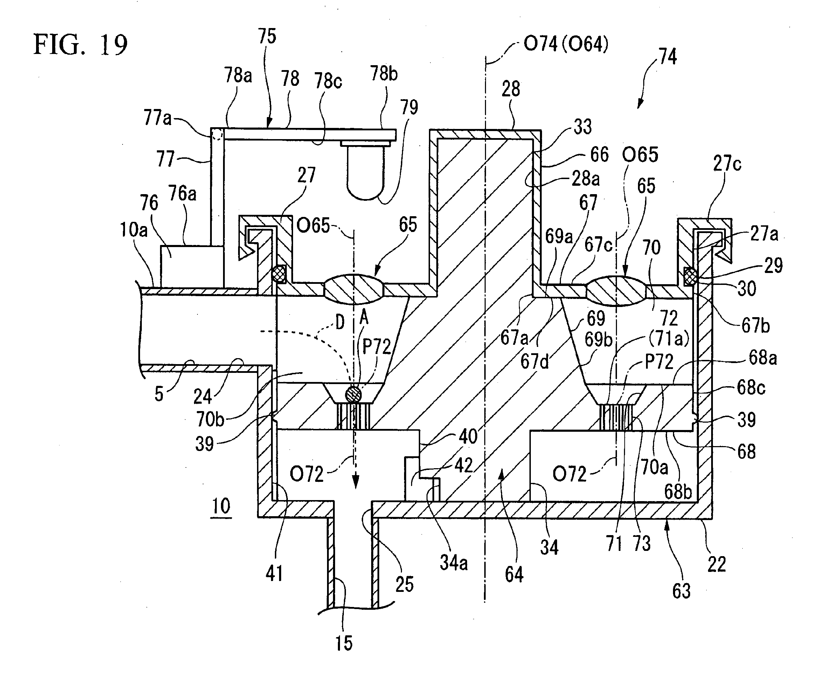

FIG. 18 and FIG. 19 show a fifth embodiment of this invention. In this embodiment, members that are the same as those used in the above described embodiments are given the same descriptive symbols and a description thereof is omitted. FIG. 16 is a perspective view showing a tissue capturing device 74 of this embodiment that is mounted on the device mounting portion 10. FIG. 17 is a cross-sectional view showing the tissue capturing device 74 of this embodiment.

As is shown in FIG. 18 and FIG. 19, the tissue capturing device 74 of this embodiment is formed by the container 63, the filter member 64 that can be housed inside the container 63, the plurality of lens members 65, and an illumination member 75. The illumination member 75 is provided with a power supply box 76, a stay 77, a plate 78, and a light emitting source 79. The power supply box 76 is provided on the top surface 10a of the device mounting portion 10. The stay 77 is a substantially plate-shaped member and protrudes vertically upward from the top surface 76a of the power supply box 76. The plate 78 is also a substantially plate-shaped member and is able to freely pivot upwards at one end portion 78a thereof. The plate 78 is fixed by a shaft to a top end portion 77a of the stay 77 such that another end portion 78b of the plate 78 faces towards the center axis 074 of the tissue capturing device 74. The light emitting source 79 is provided such that it can emit light vertically downward onto a bottom surface 78c of the plate 78. More specifically, when the tissue capturing device 74 is mounted on the device mounting portion 10, and the plate 78 is pivoted so as to be horizontal, the light emitting source 79 is able to illuminate the tissue capturing surface 72 that is positioned on a straight line connecting the intake aperture 24 and the center axis 074 of the tissue capturing device 74. In addition, a switch (not shown) is provided between the light emitting source 79 and the power supply box 76 so that light can be turned on and off as is required. Moreover, the light emitting source 79 is, for example, an LED and the color of the light emitted may be any color, but is preferably white.

In the tissue capturing device 74 of this embodiment, because the illumination member 75 is provided at a position where the biological tissue A that has been transported via the intake aperture 24 is observed, confirmation of the presence or otherwise of the biological tissue A captured on the tissue capturing surface 72 as well as of the condition thereof is further simplified.

### (Sixth embodiment)

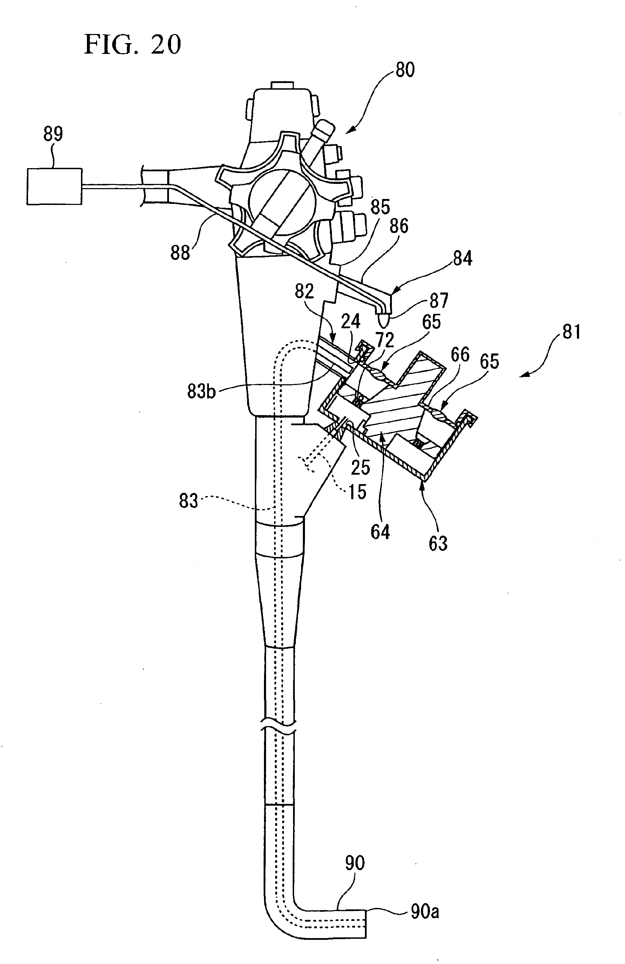

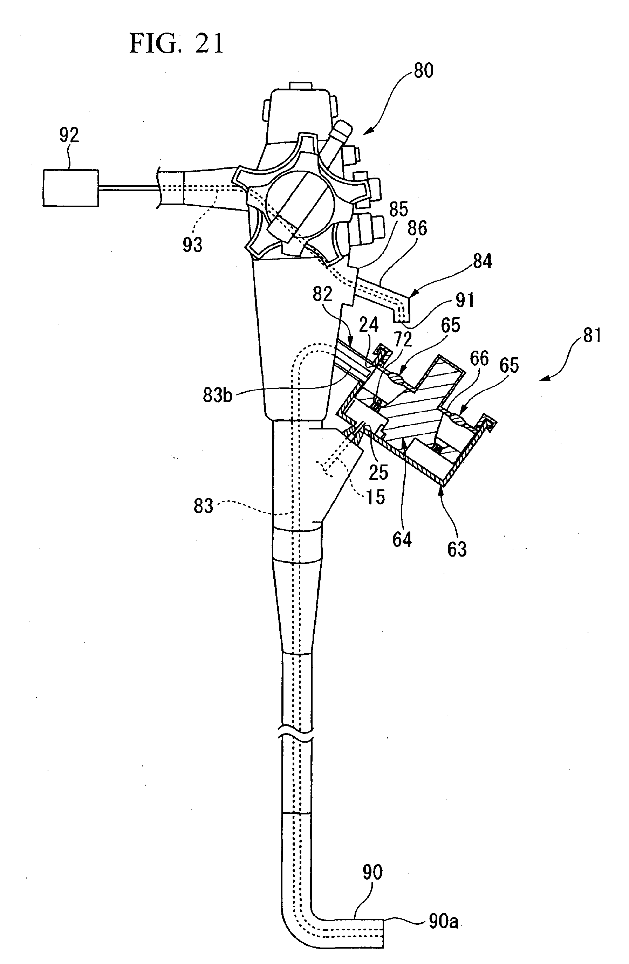

FIG. 20 and FIG. 21 show a sixth embodiment of this invention. In this embodiment, members that are the same as those used in the above described embodiments are given the same descriptive symbols and a description thereof is omitted. FIG. 20 is an overall view showing a tissue capturing device 81 mounted on an endoscope 80. FIG. 21 shows a variant example thereof.

As is shown in FIG. 20, in this embodiment, a tissue capturing device 81 is mounted on an endoscope 80. The endoscope 80 is provided with a device mounting portion 82 on which the tissue capturing device 81 can be mounted, a forceps channel 83 that is connected to the intake aperture 24 as a fluid intake tube when the tissue capturing device 81 is connected to the endoscope 80, the fluid outlet tube 15 that is connected to the outlet aperture 25, and a bracket mounting portion 85 on which an illumination member 84 of the tissue capturing device 81 can be mounted. The fluid outlet tube 15 is connected to a suction source (not shown). The tissue capturing device 81 is formed by the container 63, the filter member 64 that can be housed inside the container 63, the plurality of lens members 65, and the illumination member 84. The illumination member 84 is formed by a bracket 86 that can be mounted on the bracket mounting portion 85 of the endoscope 80, and a light emitting source 87 that is provided on a distal end portion 86a of the bracket 86. The light emitting source 87 is provided at a position where it is able to illuminate the tissue capturing surface 72 inside the container 63 from above the lid 66 of the tissue capturing device 81 when the tissue capturing device 81 has been mounted. Moreover, the light emitting source 87 is able to move freely by means of the bracket 86 so that it does not create an obstruction when the filter member 64 of the tissue capturing device 81 is being removed. The light emitting source 79 is, for example, an LED and the color of the light emitted may be any color, but is preferably white. A conducting wire 88 is connected to the light emitting source 87 and is also connected to a power supply unit 89. The conducting wire 88 may be located either inside or outside the endoscope 80.

Next, an operation of the endoscope 80 and the tissue capturing device 81 of this invention will be described. Firstly, an insertion portion 90 of the endoscope 80 is inserted by means of a distal end 90a thereof as far as a target position in a patient. Next, a treatment tool for an endoscope (not shown) such as, for example, a snare that is able to capture tissue at the target position is inserted from a base end 83b of a forceps channel 80, and tissue excision is performed. Once excision of the tissue at the target position has been completed, the endoscope treatment tool (not shown) is withdrawn from the forceps channel 83. Next, the tissue capturing device 81 is mounted on the device mounting portion 82. At this time, the intake aperture 24 of the tissue capturing device 81 is connected to the forceps channel 83, and the outlet aperture 25 of the tissue capturing device 81 is connected to the fluid outlet tube 15. In this state, if the suction source (not shown) is operated, the excised biological tissue A passes through the interior of the forceps channel 83, and is transported into the interior of the tissue capturing device 81 via the intake aperture 24. In this embodiment as well, in the same way as is described above, only the biological tissue A, which is in solid form, is captured on the tissue capturing surface 72, and it is possible to observe an enlargement of the biological tissue A using the lens member 65 facing it. Moreover, because the illumination member 84 is provided, it becomes even easier to make an observation.

An LED is used for the light emitting source 87 of the illumination member 84 of this embodiment, however, it is also possible to use a different light emitting source. FIG. 21 shows a variant example of this embodiment in which, instead of an LED, a fiber light guide 93 is used for a light emitting source 91 and in which, instead of the power supply unit 89, a light source device 92 is provided. Moreover, in this embodiment, the forceps channel 83 is used both in order to insert a treatment tool for an endoscope (not shown) and also in order to mount the tissue capturing device 81 thereon and suction the biological tissue A, however, it is also possible to provide a separate channel that is used solely for suctioning in the endoscope 80.

### INDUSTRIAL APPLICABILITY

This invention is used in tissue capturing devices that capture biological tissue which has been suctioned and recovered from inside a patient. In addition, the present invention is used in a treatment tool for an endoscope and an endoscope that are provided with this tissue capturing device and are used to perendoscopically collect biological tissue from within a patient.

## Claims

1. A tissue capturing device comprising:
a container that has an intake aperture that is connected to a fluid intake tube that transports biological tissue that has been collected by a treatment portion of a treatment tool for an endoscope and has been mixed with a liquid, and an outlet aperture that is connected to a fluid outlet tube that discharges the fluid that has been separated from the biological tissue;
a filter member that forms a screen between the intake aperture and the outlet aperture inside the container, and in which tissue capturing surfaces that are able to capture the biological tissue are formed on the intake aperture side, and in which a plurality of micro through holes that are in communication with the outlet aperture side and through which only the liquid is able to pass are formed in the tissue capturing surfaces; and
a lens member that is provided at a position inside the container where it makes it possible to observe an enlargement of the tissue capturing surfaces.

2. The tissue capturing device according to claim 1, wherein
the container is a substantially circular cylinder-shaped member that has the intake aperture in a top portion thereof and has the outlet aperture in a bottom portion thereof, and the filter member is a substantially circular member that corresponds to the shape of the container, and a plurality of capture spaces are formed by a plurality of partition walls that are provided on the intake aperture side inside the container extending radially outwards from a center axis of the filter member, and one of the tissue capturing surfaces is formed in each one of the capture spaces, and the intake aperture is able to correspond selectively to any one of the capture spaces.

3. The tissue capturing device according to claim 2, wherein
a plurality of the lens members are provided so as to correspond to a plurality of provided tissue capturing surfaces.

4. The tissue capturing device according to claim 1, wherein
the container is provided with a case in which a removal aperture for removing the filter member is provided, and
a lid that can be removably fitted onto the removal aperture.

5. The tissue capturing device according to claim 4, wherein
a pair of engaging portions that are able to be engaged with each other are provided in the lid and the filter member, and in a state in which the pair of engaging portions are engaged with each other, the lid and the filter member are able to be removed from the case of the container.

6. The tissue capturing device according to claim 1, wherein
the lens member is provided at a position facing the tissue capturing surfaces.

7. The tissue capturing device according to claim 1, wherein
a bowl-shaped recessed portion that is large enough to capture the biological tissue is formed on the intake aperture side of the filter member, and the tissue capturing surface is provided in a bottom surface of the recessed portion.

8. The tissue capturing device according to claim 1, wherein
there is provided an illumination member that illuminates the tissue capturing surface.

9. A treatment tool for an endoscope comprising:
the tissue capturing device according to claim 1;
the fluid intake tube that has a first distal end portion that is able to suction a target position inside a patient, and a first base end portion that is able to be connected to the intake aperture of the tissue capturing device; and
the fluid outlet tube that has a second distal end portion that is able to be connected to the outlet aperture of the tissue capturing device, and a second base end portion that is connected to a suction source.

10. An endoscope comprising:
the tissue capturing device according to claim 1;
the fluid intake tube that has a first distal end portion that is able to suction a target position inside a patient, and a first base end portion that is able to be connected to the intake aperture of the tissue capturing device; and
the fluid outlet tube that has a second distal end portion that is able to be connected to the outlet aperture of the tissue capturing device, and a second base end portion that is connected to a suction source.

## Patentansprüche

1. Gewebeauffangvorrichtung, die umfasst:
einen Behälter, der eine Einlassöffnung, die mit einem Fluideinlassschlauch verbunden ist, der biologisches Gewebe transportiert, das durch einen Behandlungsabschnitt eines Behandlungsinstruments gesammelt wurde und das mit einer Flüssigkeit gemischt wurde, und eine Auslassöffnung hat, die mit einem Fluidauslassschlauch verbunden ist, der das Fluid, das von dem biologischen Gewebe getrennt wurde, abführt;
ein Filterelement, das ein Sieb innerhalb des Behälters zwischen der Einlassöffnung und der Auslassöffnung bildet und in dem Gewebeauffangoberflächen, die dazu eingerichtet sind, biologisches Gewebe zu aufzufangen, auf der Seite der Einlassöffnung ausgebildet sind, und in dem eine Mehrzahl von Mikrodurchlassöffnungen, die in Verbindung mit der Auslassöffnungsseite sind und durch die nur die Flüssigkeit hindurchtreten kann, in den Gewebeauffangoberflächen ausgebildet sind; und
ein Linsenelement, das an einer Position in dem Behälter vorgesehen ist, die es ermöglicht, eine Vergrößerung der Gewebeauffangoberflächen zu beobachten.

2. Gewebeauffangvorrichtung gemäß Anspruch 1, wobei der Behälter ein im Wesentlichen kreiszylinderförmiges Element ist, das die Einlassöffnung in seinem oberen Abschnitt und die Auslassöffnung in seinem unteren Abschnitt hat, und das Filterelement ein im Wesentlichen kreisförmiges Element ist, das der Form des Behälters entspricht und eine Mehrzahl von Auffangräumen durch eine Mehrzahl von Trennwänden gebildet wird, die auf der Seite der Einlassöffnung innerhalb des Behälters vorgesehen sind und sich von einer Zentralachse des Filterelements radial nach außen erstrecken, und wobei die Einlassöffnung dazu eingerichtet ist, selektiv einem der Entnahmeräume zu entsprechen.

3. Gewebeauffangvorrichtung gemäß Anspruch 2, wobei eine Mehrzahl von Linsenelementen dazu vorgesehen ist, einer Mehrzahl von vorgesehenen Gewebeauffangoberflächen zu entsprechen.

4. Gewebeauffangvorrichtung gemäß Anspruch 1, wobei
der Behälter mit einem Gehäuse, in dem eine Entfernungsöffnung zum Entfernen des Filterelements vorgesehen ist, und
einem Deckel versehen ist, der entfernbar auf der Entfernungsöffnung angebracht werden kann.

5. Gewebeauffangvorrichtung gemäß Anspruch 4, wobei
ein Paar von Eingriffsabschnitten, die miteinander in Eingriff bringbar sind, in dem Deckel und dem Filterelement vorgesehen sind, und in einem Zustand, in dem das Paar von Eingriffsabschnitten miteinander in Eingriff gebracht ist, der Deckel und das Filterelement von dem Gehäuse des Behälters gelöst werden kann.

6. Gewebeauffangvorrichtung gemäß Anspruch 1, wobei
das Linsenelement an einer Position vorgesehen ist, die den Gewebeauffangoberflächen gegenüberliegt.

7. Gewebeauffangvorrichtung gemäß Anspruch 1, wobei
ein schalenförmiger vertiefter Abschnitt, der groß genug ist, um das biologische Gewebe aufzufangen, auf der Seite der Einlassöffnung des Filterelements ausgebildet ist und die Gewebeauffangoberfläche in einer Bodenfläche des vertieften Abschnitts vorgesehen ist.

8. Gewebeauffangvorrichtung gemäß Anspruch 1, wobei
ein Beleuchtungselement, das die Gewebeauffangoberfläche beleuchtet, vorgesehen ist.

9. Behandlungsinstrument für ein Endoskop, das umfasst:
die Gewebeauffangvorrichtung gemäß Anspruch 1;
den Fluideinlassschlauch, der einen ersten distalen Endabschnitt, der dazu in der Lage ist, eine Zielposition innerhalb eines Patienten anzusaugen, und einen ersten Basisendabschnitt hat, der dazu in der Lage ist, mit der Einlassöffnung der Gewebeauffangvorrichtung verbunden zu werden; und
den Fluidauslassschlauch, der einen zweiten distalen Endabschnitt, der dazu in der Lage ist, mit der Auslassöffnung der Gewebeauffangvorrichtung verbunden zu werden, und einen zweiten Basisendabschnitt hat, der mit einer Saugquelle verbunden ist.

10. Endoskop, das umfasst:
die Gewebeauffangvorrichtung gemäß Anspruch 1;
den Fluideinlassschlauch, der einen ersten distalen Endabschnitt, der dazu in der Lage ist, eine Zielposition innerhalb eines Patienten anzusaugen, und einen ersten Basisendabschnitt hat, der dazu in der Lage ist, mit der Einlassöffnung der Gewebeauffangvorrichtung verbunden zu werden; und
den Fluidauslassschlauch, der einen zweiten distalen Endabschnitt, der dazu in der Lage ist, mit der Auslassöffnung der Gewebeauffangvorrichtung verbunden zu werden, und einen zweiten Basisendabschnitt hat, der mit einer Saugquelle verbunden ist.

## Revendications

1. Dispositif de capture de tissu comprenant :
un récipient qui comporte une ouverture d'entrée qui est connectée à un tube d'entrée de fluide qui transporte un tissu biologique qui a été collecté par une partie de traitement d'un outil de traitement pour endoscope et qui a été mélangé avec un liquide, et une ouverture de sortie qui est connectée à un tube de sortie de fluide qui évacue le liquide qui a été séparé du tissu biologique ;
un élément de filtre qui forme un écran entre l'ouverture d'entrée et l'ouverture de sortie à l'intérieur du récipient, et dans lequel des surfaces de capture de tissu qui sont capables de capturer le tissu biologique sont formées sur le côté de l'ouverture d'entrée, et dans lequel une pluralité de micro trous traversants qui sont en communication avec le côté de l'ouverture de sortie et à travers lesquels seul le liquide est capable de passer sont formés dans les surfaces de capture de tissu ; et
un élément de lentille qui est prévu au niveau d'une position à l'intérieur du récipient à laquelle il rend possible d'observer un agrandissement des surfaces de capture de tissu.

2. Dispositif de capture de tissu selon la revendication 1, dans lequel
le récipient est un élément de forme cylindrique sensiblement circulaire qui comporte l'ouverture d'entrée dans une partie supérieure de celui-ci et comporte l'ouverture de sortie dans une partie inférieure de celui-ci, et l'élément de filtre est un élément sensiblement circulaire qui correspond à la forme du récipient, et une pluralité d'espaces de capture sont formés par une pluralité de parois de séparation qui sont prévues sur le côté de l'ouverture d'entrée à l'intérieur du récipient s'étendant radialement vers l'extérieur depuis un axe central de l'élément de filtre, et l'une des surfaces de capture de tissu est formée dans chacun des espaces de capture, et l'ouverture d'entrée est capable de correspondre sélectivement à l'un quelconque des espaces de capture.

3. Dispositif de capture de tissu selon la revendication 2, dans lequel
une pluralité d'éléments de lentille sont prévus de façon à correspondre à une pluralité de surfaces de capture de tissu prévues.

4. Dispositif de capture de tissu selon la revendication 1, dans lequel
le récipient est muni d'un boîtier dans lequel une ouverture de retrait destinée à retirer l'élément de filtre est prévue, et
un couvercle qui peut être ajusté de manière amovible sur l'ouverture de retrait.

5. Dispositif de capture de tissu selon la revendication 4, dans lequel
des parties d'engagement formant une paire qui sont capables de s'engager l'une dans l'autre sont prévues dans le couvercle et l'élément de filtre, et dans un état dans lequel les parties d'engagement formant une paire sont engagées l'une dans l'autre, le couvercle et l'élément de filtre sont capables d'être retirés du boîtier du récipient.

6. Dispositif de capture de tissu selon la revendication 1, dans lequel
l'élément de lentille est prévu au niveau d'une position en regard des surfaces de capture de tissu.

7. Dispositif de capture de tissu selon la revendication 1, dans lequel
une partie évidée en forme de bol qui est suffisamment grande pour capturer le tissu biologique est formée sur le côté de l'ouverture d'entrée de l'élément de filtre, et la surface de capture de tissu est prévue dans une surface de fond de la partie évidée.

8. Dispositif de capture de tissu selon la revendication 1, dans lequel
il est prévu un élément d'éclairage qui éclaire la surface de capture de tissu.

9. Outil de traitement pour un endoscope comprenant :
le dispositif de capture de tissu selon la revendication 1 ;
le tube d'entrée de fluide qui comporte une première partie d'extrémité distale qui est capable d'aspirer une position cible à l'intérieur d'un patient, et une première partie d'extrémité de base qui est capable d'être connectée à l'ouverture d'entrée du dispositif de capture de tissu ; et
le tube de sortie de fluide qui comporte une deuxième partie d'extrémité distale qui est capable d'être connectée à l'ouverture de sortie du dispositif de capture de tissu, et une deuxième partie d'extrémité de base qui est connectée à une source d'aspiration.

10. Endoscope comprenant :
le dispositif de capture de tissu selon la revendication 1 ;
le tube d'entrée de fluide qui comporte une première partie d'extrémité distale qui est capable d'aspirer une position cible à l'intérieur d'un patient, et une première partie d'extrémité de base qui est capable d'être connectée à l'ouverture d'entrée du dispositif de capture de tissu ; et
le tube de sortie de fluide qui comporte une deuxième partie d'extrémité distale qui est capable d'être connectée à l'ouverture de sortie du dispositif de capture de tissu, et une deuxième partie d'extrémité de base qui est connectée à une source d'aspiration.
